Europäisches Patentamt

European Patent Office

Office européen des brevets

(19)

(11) Veröffentlichungsnummer: **0 001 078**
**B1**

(12) **EUROPÄISCHE PATENTSCHRIFT**

(45) Veröffentlichungstag der Patentschrift: **15.07.81**

(21) Anmeldenummer: **78100759.6**

(22) Anmeldetag: **28.08.78**

(51) Int. Cl.³: **C 07 D 301/10,**
**B 01 J 23/96**

(54) Verfahren zur Verbesserung der Wirksamkeit von gebrauchten Silber-Trägerkatalysatoren.

(30) Priorität: **08.09.77 DE 2740480**

(43) Veröffentlichungstag der Anmeldung:
**21.03.79 Patentblatt 79/6**

(45) Bekanntmachung des Hinweises auf die Patenterteilung:
**15.07.81 Patentblatt 81/28**

(84) Benannte Vertragsstaaten:
**BE DE FR GB NL SE**

(56) Entgegenhaltungen:
**DE - A - 2 209 392**
**DE - A - 2 611 856**
**DE - A - 2 746 976**
**FR - A - 837 792**
**US - A - 2 404 438**
**US - A - 4 033 903**

(73) Patentinhaber: **HOECHST AKTIENGESELLSCHAFT**
**Postfach 80 03 20**
**D-6230 Frankfurt/Main 80 (DE)**

(72) Erfinder: **Rebsdat, Siegfried, Dr.**
**Trenbeckstrasse 24**
**D-8261 Burg (DE)**
Erfinder: **Mayer, Sigmund**
**Watzmannring 19**
**D-8261 Burgkirchen/Alz (DE)**
Erfinder: **Alfranseder, Josef**
**Hauptstrasse 31**
**D-8261 Markt1/Inn (DE)**
Erfinder: **Riedl, Josef, Dr.**
**Kantstrasse 53**
**D-8261 Burgkirchen/Alz (DE)**

Courier Press, Leamington Spa, England.

Verfahren zur Verbesserung der Wirksamkeit von gebrauchten Silber-Trägerkatalysatoren

Die Erfindung betrifft ein Verfahren zur Verbesserung der Wirksamkeit von Silber-Trägerkatalysatoren für die Direktoxidation von Äthylen zu Äthylenoxid mit molekularem Sauerstoff oder Luft, wobei auf den für die Direktoxidation gebrauchten Katalysator 1 bis 1000 Teile je 1 Million Teile Katalysator, Cäsium, Rubidium oder eine Mischung davon aufgebracht wird.

Zur Herstellung von Äthylenoxid durch Oxidation von Äthylen mit Sauerstoff oder Luft werden Silberkatalysatoren eingesetzt, deren Herstellung seit langem bekannt beschrieben ist. Eine ganze Reihe großtechnischer Anlagen zur Herstellung von Äthylenoxid arbeiten nach dem Silberkatalysator-Verfahren. Dabei wird üblicherweise nur ein Bruchteil des eingesetzten Äthylens umgesetzt. Das umgesetzte Äthylen wird an dem mit Silber imprägnierten Trägermaterial mit Sauerstoff zum überwiegenden Teil in Äthylenoxid überführt, der Rest wird praktisch vollständig in Kohlendioxid und Wasser verwandelt.

Im Laufe der Zeit sind verschiedene Silberkatalysatoren entwickelt worden, und zwar mit dem Ziel, die Selektivität in bezug auf die bevorzugte Bildung von Äthylenoxid zu erhöhen und die Bildung von $CO_2$ und Wasser zurückzudrängen.

Bei steigenden Rohstoffpreisen und zunehmender Rohstoffverknappung kommt einer erhöhten Selektivität der Katalysatoren besondere wirtschaftliche Bedeutung zu. So ist es in den letzten Jahren gelungen, Silberkatalysatoren zu entwickeln, deren Selektivität bei bis zu 75% Äthylenoxid gegenüber älteren Typen mit nur 65 bis 70% Selektivität liegt. Solche Katalysatoren — wie sie beispielsweise in der DE—SO 23 00 512 beschrieben sind — werden dadurch erhalten, daß man auf ein inertes Trägermaterial, wie beispielsweise $Al_2O_3$, gleichzeitig mit dem Silber 0,0004 bis 0,0027 g-Äquivalente einer Kalium-, Rubidium- oder Cäsiumverbindung pro Kilogramm Katalysator aus wäßriger Lösung aufbringt.

Andererseits ist auch bekannt, daß Silberkatalysatoren im Lauf der Zeit an Selektivität verlieren und nach einigen Jahren Gebrauch durch neuen Katalysator ersetzt werden müssen. Der Austausch eines "ermüdeten" Katalysators gegen einen neuen in großtechnischen Anlagen ist — abgesehen von den Materialkosten — außerordentlich zeitraubend und arbeitsintensiv; er verursacht zudem Produktionsausfall und hohe Kosten. Demzufolge stellt sich die Aufgabe, ob es möglich ist, ermüdete Katalysatoren durch eine einfache Behandlung wieder in ihrer Selektivität zu verbessern, um den Austausch gegen einen neuen Katalysator zu vermeiden bzw. möglichst weit hinauszuschieben.

Ein solches Verfahren wird in den beiden deutschen Patentschriften 25 19 599 und 26 11 856 beschrieben. Hierbei wird ein für die Direktoxidation von Äthylen zu Äthylenoxid mit molekularem Sauerstoff oder Luft in Gebrauch gewesener Silber-Trägerkatalysator mit einer Cäsium- und/oder Rubidium-nitrat-Lösung in gegebenenfalls wasserhaltigem aliphatischem Alkohol getränkt, wobei auf den gebrauchten Katalysator 1 bis 1000 Gewichtsteile je 1 Million Gewichtsteile (mg/kg) Katalysator an Cäsium, Rubidium oder eine Mischung aus beiden aufgebracht wird. Der so behandelte Katalysator weist eine erheblich verbesserte Selektivität auf.

In diesen beiden Patentschriften wird also die gleichzeitige Behandlung eines gebrauchten Silber-Trägerkatalysators mit Cäsium- und/oder Rubidiumverbindungen und einem aliphatischen Alkohol beschrieben.

Ein weiteres Verfahren zur Verbesserung der Wirksamkeit von gebrauchten Silber-Trägerkatalysatoren ist aus der deutschen Offenlegungsschrift 27 46 976 bekannt. Dabei wird der gebrauchte Silber-Trägerkatalysator zunächst mit Wasser oder einem Gemisch aus Wasser und einem mit Wasser mischbaren organischen Lösungsmittel gewaschen, worauf 0,00004 bis 0,008 Gramm-Äquivalent pro Kilogramm (bezogen auf den gesamten Katalysator) Ionen eines oder mehrerer der Alkalimetalle Natrium, Kalium, Rubidium oder Cäsium aufgebracht werden.

In dieser Offenlegungsschrift wird mehrmals darauf hingewiesen, daß das Waschen mit Wasser oder einem Gemisch aus Wasser und einem mit Wasser mischbaren organischen Lösungsmittel vorgenommen werden muß.

In der deutschen Offenlegungsschrift 22 09 392 ist ferner ein Verfahren zur Herstellung und Aktivierung von neuen Silber-Trägerkatalysatoren beschrieben, womit eine bessere Anfangwirkung des neu hergestellten Katalysators erreicht werden soll. Dieses bekannte Verfahren bezieht sich also auf die Herstellung von neuen, das heißt frisch hergestellten und noch nicht gebrauchten Silber-Trägerkatalysatoren. Dabei wird, neben mehreren anderen Verfahrensmaßnahmen, auch ein Waschen des frisch bereiteten Katalysators mit niederen aliphatischen Alkoholen vorgenommen. Dieses Waschen hat offensichtlich den Zweck, Lösungsmittel wie Äthylendiamin und Monoäthanolamin, und andere Verunreinigungen, die beim Aufbringen der Silbersalze auf das poröse Trägermaterial zurückbleiben, wegzuwaschen, da sie die Anfangswirkung des neuen Katalysators offensichtlich besonders herabsetzen würden.

Auf einem gebrauchten Silber-Trägerkatalysator sind derartige Verunreinigungen nicht mehr vorhanden, da sie, nach mehr oder weniger langem Gebrauch des Katalysators zur Produktion von Äthylenoxid, die bei Temperaturen von 220 bis 260°C erfolgt, zwangsläufig entfernt worden sind.

Es wurde nun überraschenderweise festgestellt, daß die Verbesserung der Selektivität von gebrauchten Silber-Trägerkatalysatoren durch Aufbringen von 1 bis 1000 mg/kg an Cäsium und/oder Rubidium auf den gebrauchten Katalysator noch weiter gesteigert werden kann, wenn man ihn vor dem Aufbringen von 1 bis 1000 mg/kg an Cäsium und/oder Rubidium einer Wäschebehandlung mit einer inerten organischen Flüssigkeit unterwirft.

Gefunden wurde demnach ein Verfahren zur Verbesserung der Wirksamkeit von Silber-Trägerkatalysatoren für die Direktoxidation von Äthylen zu Äthylenoxid mit molekularem Sauerstoff oder Luft, wobei auf den für die Direktoxidation gebrauchten Katalysator 1 bis 1000 Teile je 1 Million Teile Katalysator, Cäsium, Rubidium oder eine Mischung davon aufgebracht wird, dadurch gekennzeichnet, daß vor dem Aufbringen der gebrauchte Katalysator mit einer inerten organischen Flüssigkeit gewaschen wird.

Unter den organischen Flüssigkeiten (einzeln oder in Mischung) kommen beispielsweise in Betracht aliphatische, alicyclische oder aromatische Kohlenwasserstoffe; aliphatische, alicyclische oder aromatische Äther, Alkohole und Ketone; aliphatische oder aromatische Säuren wie Essigsäure, Propionsäure; Ester, Amine, Amide oder Aldehyde wie Acetaldehyd, Benzaldehyd. Schwefelhaltige Flüssigkeiten werden, da sie im allgemeinen Gift für Silberkatalysatoren darstellen, in der Regel nicht in Betracht kommen.

Es hat sich als zweckmäßig erwiesen, solche organischen Flüssigkeiten zu verwenden, deren Siedepunkt nicht höher als 350°C, vorzugsweise nicht höher als 250°C, insbesondere nicht höher als 150°C und nicht tiefer als 0°C, vorzugsweise nicht tiefer als 15°C liegt.

Bevorzugt sind solche inerte organische Flüssigkeiten, die einen Siedepunkt von 20 bis 150°C aufweisen.

Unter den organischen Flüssigkeiten werden bevorzugt eingesetzt: Aliphatische, alicyclische oder aromatische Kohlenwasserstoffe, vorzugsweise solche mit 5 bis 10 C-Atomen wie Pentan, Hexan, Heptan, Petroläther (40 bis 70°C), Leichtbenzin (70 bis 90°C), Cyclohexan, Benzol, Toluol, Xylol, aliphatische, alicyclische oder aromatische Äther, vorzugsweise aliphatische oder alicyclische Äther mit 3 bis 10 C-Atomen wie Diäthyläther, Methylisopropyläther, Äthylenglykolmethyläther, Äthylenglykoldimethyläther, Tetrahydrofuran, Dioxan, aliphatische, alicyclische oder aromatische Ketone, vorzugsweise aliphatische Ketone mit 3 bis 10 C-Atomen wie Aceton, Äthylpropylketon, Methyläthylketon, Dipropylketon, aliphatische Ester mit 3 bis 10 C-Atomen wie Methylacetat, Äthylacetat, Butylacetat und aliphatische, alicyclische oder aromatische Alkohole, vorzugsweise aliphatische oder alicyclische Alkohole mit 1 bis 6 C-Atomen, insbesondere aliphatische Alkohole mit 1 bis 3 C-Atomen, wie Methanol, Äthanol, Propanol und Isopropanol, allein oder in Mischung untereinander sowie in Mischung mit Wasser.

Besonders bevorzugte organische Flüssigkeiten sind aliphatische Kohlenwasserstoffe mit 5 bis 10 C-Atomen, Petroläther, Cyclohexan, Benzol, aliphatische Äther mit 4 bis 8 C-Atomen, Dioxan, Tetrahydrofuran, aliphatische Ketone mit 3 bis 6 C-Atomen und aliphatische Alkohole mit 1 bis 3 C-Atomen allein oder in Mischung untereinander sowie in Mischung mit Wasser.

Das Waschen des gebrauchten Katalysators kann durch verschiedene Arbeitsweisen vorgenommen werden. Es kommt dabei lediglich darauf an, daß der Katalysator mit der Waschflüssigkeit in Berührung gebracht und anschließend davon größtenteils abgetrennt wird. Die Behandlung (Waschen) des Katalysators mit der Waschflüssigkeit kann auch mehrmals wiederholt werden. Die Zeit, innerhalb welcher der Katalysator mit der Waschflüssigkeit in Berührung ist, ist nicht kritisch; sie kann von wenigen Minuten bis zu mehreren Stunden oder Tagen reichen und liegt in der Regel bei 5 Minuten bis 5 Tagen, vorzugsweise bei 30 Minuten bis 48 Stunden, insbesondere bei 1 bis 5 Stunden. Das Waschen kann dadurch erfolgen, daß der Katalysator in einem Behälter (Gefäß) mit der Waschflüssigkeit überschüttet und anschließend von der Waschflüssigkeit durch Zentrifugieren, Filtrieren, Abnutschen oder einfach Abgießen (Abdenkantieren) abgetrennt wird. Dabei ist es auch möglich, den übergossenen Katalysator mehr oder weniger lang, beispielsweise über die obengenannte Zeit (30 Minuten bis 48 Stunden, vorzugsweise 1 bis 5 Stunden), gegebenenfalls unter Rühren, stehenzulassen und erst anschließend die Waschflüssigkeit abzutrennen. In der Regel genügt es, um den Effekt gemäß Erfindung zu erreichen, wenn der Waschvorgang einmal vorgenommen wird. Es kann jedoch von Vorteil sein, den Waschvorgang mehrere Male zu wiederholen, vorzugsweise wird er 1- bis 5mal, insbesondere 2- bis 3mal durchgeführt, wobei vorteilhafterweise jeweils eine frische (neue, noch nicht gebrauchte) Waschflüssigkeit verwendet wird. Neben der geschilderten Arbeitsweise kann das Waschen auch dadurch vorgenommen werden, daß der beispielsweise in einem oder mehreren Rohren als Festbett angeordnete Katalysator mit der Waschflüssigkeit übergossen wird, wobei die Waschflüssigkeit an einem Ende der Rohre aufgegeben und am anderen Ende der Rohre ablaufen gelassen wird. Auch dieser Waschvorgang kann mehrere Male wiederholt werden und wird vorzugsweise 1- bis 5mal durchgeführt, wobei zweckmäßigerweise jeweils eine frische (noch nicht gebrauchte) Waschflüssigkeit eingesetzt wird. Es kann von Vorteil sein, beim Übergießen des Katalysators (ein oder mehrere Male) die Waschflüssigkeit nicht gleich ablaufen zu lassen, sondern sie für einige Zeit, vorzugsweise für 30 Minuten bis zu 48 Stunden, im Rohr oder in den Rohren zu belassen, worauf der Abfluß freigegeben wird. Diese Art des Waschens, nämlich Fluten, wird man bevorzugt in Großanlagen anwenden, wo der gebrauchte Katalysator bereits in den Rohren des Reaktors als Festbett vorliegt. Dabei wird der mit dem Katalysator gefüllte Reaktor mit der Waschflüssigkeit übergossen und die abfließende Waschflüssigkeit am Reaktorausgang aufgefangen.

Es ist nicht erforderlich, den gesamten gebrauchten Katalysator zu waschen. Es kann der im Sinne der Erfindung angestrebte Effekt auch schon erreicht werden, wenn nur ein Teil, beispielsweise 30 bis 70% des gebrauchten (gesamt vorliegenden) Katalysators gewaschen wird und der andere Teil nicht gewaschen wird.

Die Menge an Waschflüssigkeit (für ein- oder mehrmalige Wäsche) richtet sich nach der zu waschenden Katalysatormenge und ist natürlich so zu bemessen, daß der Katalysator mit der Flüssigkeit in Berührung kommt. Zweckmäßigerweise beträgt die Menge an Waschflüssigkeit (für einen einmaligen Waschvorgang) in Gewichtsteilen oder Volumenteilen mindestens etwa 1/3 der Menge an Katalysator in Volumenteilen, der zu waschen ist; vorzugsweise werden etwa gleiche bis fünffache, insbesondere zwei- bis dreifache Mengen an Waschflüssigkeit eingesetzt.

Die Temperatur (des Katalysators und der Waschflüssigkeit) während des Waschens ist nicht kritisch und ist mehr von praktischen Gesichtspunkten bestimmt. Sie liegt zweckmäßigerweise unterhalb der Verdampfungstemperatur der Waschflüssigkeit und in der Regel bei 10 bis 100, vorzugsweise bei 20 bis 50°C. Ebenfalls wird von praktischen Gesichtspunkten bestimmt, ob der Waschvorgang drucklos oder unter Druck durchgeführt wird. In der Regel wird man das Waschen drucklos durchführen. Sollte andererseits unter Druck gewaschen werden, so liegt er zweckmäßigerweise bei 1 bis 20 atü, vorzugsweise bei 1 bis 5 atü.

Nach dem Waschen erfolgt erfindungsgemäß das Aufbringen von 1 bis 1000 mg/kg Cäsium Rubidium oder einer Mischung dieser beiden (wobei das Mengenverhältnis beliebig sein kann).

Das Aufbringen von Cäsium und/oder Rubidium auf den gewaschenen Katalysator, der zweite Verfahrensschritt des erfindungsgemäßen Verfahrens, kann direkt nach dem ersten Verfahrensschritt, dem Waschen, vorgenommen werden oder auch erst nachdem der Katalysator einer Trocknung unterworfen worden ist, wobei vom Waschen (nach Abtrennen bzw. Abfließen der Waschflüssigkeit) gegebenenfalls verbliebene Flüssigkeitsreste entfernt werden.

Die Trocknung kann beispielsweise durch Hindurchleiten eines Inertgases und/oder durch Erwärmen des Katalysators erreicht werden, wobei zur Beschleunigung des Trocknungsvorganges auch Unterdruck angewendet werden kann. Die bei der Erwärmung angewendete Temperatur ist nicht kritisch, sie richtet sich nach der eingesetzten Waschflüssigkeit und wird, sofern bei Atmosphärendruck getrocknet wird, etwa dem Siedepunkt der Waschflüssigkeit entsprechen. Zweckmäßige Trocknungstemperaturen sind beispielsweise etwa 20 bis 250°C, vorzugsweise 50 bis 150°C. Wenn man mit Unterdruck trocknet, kann auch schon bei Zimmertemperatur, das heißt bei 15 bis 25°C oder beispielsweise bei 25 bis 80°C, eine vollständige Verdampfung der am Katalysator verbliebenen Waschflüssigkeit, nachdem der größte Teil bereits abgetrennt wurde, erreicht werden. Erwärmen und gleichzeitiges Überleiten eines Inertgases beschleunigt das Trocknen ebenfalls. Als Inertgas werden zweckmäßigerweise unbrennbare, die Verbrennung nicht fördernde Gase wie Stickstoff oder Kohlendioxid verwendet. Sofern Zündquellen ausgeschaltet werden und/oder ein großer Überschuß des Gases verwendet wird, der mit den flüchtigen Stoffen keine zündfähigen Gemische bildet, können auch andere Gase, insbesondere Luft, verwendet werden.

Für das Aufbringen von Cäsium und/oder Rubidium auf den gewaschenen (getrockneten oder nicht getrockneten) Katalysator können verschiedene Arbeitsweisen gewählt werden; wichtig ist lediglich, daß die angegebenen Mengen von Cäsium, Rubidium oder auch Abmischungen davon auf den Katalysator (und zwar auf den gesamten gebrauchten Katalysator, also auch auf gegebenenfalls nicht gewaschene Teilmengen) aufgebracht werden.

Zweckmäßigerweise erfolgt das Aufbringen von Cäsium und/oder Rubidium durch Benetzen (Tränken, Imprägnieren) des Katalysators mit einer Imprägnierflüssigkeit, die eine oder mehrere Verbindungen von Cäsium und/oder Rubidium enthält.

Die Imprägnierflüssigkeit soll die Cäsium und/oder Rubidiumverbindungen in möglichst fein verteilter Form enthalten. Die genannten Verbindungen können in Dispersion oder Emulsion vorliegen, vorzugsweise werden sie jedoch in gelöster Form augewendet (Imprägnierlösung).

Als Lösungsmittel bzw. flüssige Phase einer Dispersion oder Emulsion können die oben als Waschflüssigkeit beschriebenen organischen Flüssigkeiten verwendet werden. Bevorzugte Lösungsmittel sind aliphatische, alicyclische oder aromatische Kohlenwasserstoffe, vorzugsweise solche mit 5 bis 10 C-Atomen, wie Pentan, Hexan, Heptan, Cyclohexan, Benzol; aliphatische, alicyclische oder aromatische Äther, vorzugsweise aliphatische oder alicyclische Äther mit 3 bis 10 C-Atomen, wie Diäthyläther, Methyläthyläther, Dipropyläther, Methylisopropyläther, Tetrahydrofuran, Dioxan;

aliphatische, alicyclische oder aromatische Ketone, vorzugsweise aliphatische Ketone mit 3 bis 10 C-Atomen, wie Aceton, Methyläthylketon, Äthylpropylketon; aliphatische oder aromatische Ester, vorzugsweise aliphatische Ester mit 3 bis 10 C-Atomen, wie Methylacetat, Äthylacetat; und aliphatische, alicyclische oder aromatische Alkohole, vorzugsweise aliphatische oder alicyclische Alkohole mit 1 bis 6 C-Atomen.

Besonders bevorzugt werden aliphatische (geradkettige oder verzweigte) Alkohole mit 1 bis 6 C-Atomen, insbesondere mit 1 bis 3 C-Atomen, wie Methanol, Äthanol, Propanol, Isopropanol.

Die organischen Lösungsmittel können für sich allein (wobei auch Mischungen untereinander möglich sind) eingesetzt werden oder in Mischung mit Wasser. Auch reines Wasser ist als Lösungsmittel verwendbar. Sofern Mischungen aus organischen Lösungsmitteln und Wasser eingesetzt werden, sind solche bevorzugt, deren Wassergehalt bis zu 40 Gew.-%, vorzugsweise bis zu 20 Gew.-%, bezogen auf Gesamtflüssigkeit, ausmacht.

Die Art der Cäsium- und/oder Rubidiumverbindungen ist für den erfindungsgemäßen Effekt nicht entscheidend. In der Regel wird die gewählte Verbindung oder die gewählten Verbindungen Cäsium und Rubidium in Gestalt der entsprechenden Kationen enthalten. In Verbindung mit welchem Rest (Anion) Cäsium und/oder Rubidium vorliegt, ist von geringer Bedeutung. Es kann sich um anorganische oder organische Reste handeln. Allerdings sollte dieser Rest nicht aus Stoffen bestehen, die insbesondere nach Behandlung mit der gasförmigen Reaktionsmischung zur Herstellung von Äthylenoxid als sogenanntes Katalysatorengift wirken. Geeignete Cäsium- und Rubidiumverbindungen sind:
Anorganische Verbindungen, vorzugsweise anorganische Salze, beispielsweise Sulphate, Nitrite, Nitrate, Chloride, Bromide, Jodide, Fluoride, Chlorate, Silikate, Carbonate, Bicarbonate und Cyanate; Hydroxide und Oxide, organische Verbindungen, vorzugsweise organische Salze, beispielsweise Formiate, Acetate, Oxalate, Malonate, Succinate, Butyrate, Laurate, Stearate, Lactate, Tartrate und Benzoate; und Alkoholate beispielsweise Methylat, Äthylat und Phenolat. Bevorzugt eingesetzt werden anorganische oder organische Salze, insbesondere die Formiate, Acetate, Carbonate, Bicarbonate, Nitrate; die Hydroxide und die Alkoholate von aliphatischen Alkoholen, vorzugsweise mit 1 bis 6, besonders mit 1 bis 3 C-Atomen.

Es können sowohl eine als auch mehrere Cäsium- oder Rubidiumverbindungen eingesetzt werden, Mischungen von Cäsium- und Rubidiumverbindungen sind ebenfalls geeignet.

Die Konzentration der Cäsium- und/oder Rubidiumverbindungen in der Imprägnierflüssigkeit ist nicht kritisch; sie wird sich im allgemeinen nach der Löslichkeit der Verbindungen richten. Entscheidend ist lediglich, daß nach dem Behandeln des Katalysators mit der Imprägnierflüssigkeit eine Konzentration von 1 bis 1000 mg/kg an Cäsium und/oder Rubidium auf dem Katalysator eingestellt ist (die Konzentrationsangabe für Cäsium oder Rubidium auf dem Katalysator bezieht sich nur auf das Cäsium oder Rubidiummetall, der Rest in der gewählten Verbindung bleibt bei dieser Angabe außer Betracht). Es empfiehlt sich, in der Imprägnierflüssigkeit (Imprägnierlösung) eine Mindestkonzentration von 0,0003 Gew.-% an Cäsium und/oder Rubidium zu nehmen. Als besonders zweckmäßig hat sich eine Konzentration von 0,005 bis 1,0 Gew.-%, vorzugsweise von 0,01 bis 0,4 Gew.-%, an Cäsium- und/oder Rubidumverbindung, bezogen auf Imprägnierlösung, erwiesen. Jedoch richtet man sich bei der Einstellung der Konzentration der Imprägnierlösung stets nach der erwünschten Konzentration an Rubidium oder Cäsium auf dem Katalysator.

Auch die Menge an Imprägnierflüssigkeit kann in weiten Grenzen variiert werden. Man wird sich dabei nach der Menge an zu behandelndem Katalysator richten, so daß alle Katalysatorteilchen auch vollständig benetzt werden. Nach oben ist somit die Menge der Imprägnierflüssigkeit von ihrer Wirkung her nicht begrenzt. Im allgemeinen wird man 75 bis 150 Vol.-% Imprägnierflüssigkeit, bezogen auf zu behandelnden Katalysator, nehmen.

Die Behandlung des gewaschenen Katalysators mit der Imprägnierflüssigkeit, um 1 bis 1000 mg/kg, vorzugsweise 3 bis 500 mg/kg Cäsium und/oder Rubidium auf den Katalysator aufzubringen, kann nach verschiedenen Arbeitsweisen durchgeführt werden. Eine zweckmäßige Arbeitsweise ist die in der DE—PS 25 19 599 beschriebene Tränkung (Benetzung, Imprägnierung), wobei der Katalysator mit der Imprägnierflüssigkeit übergossen und der überschüssige Teil vom Katalysator abgetrennt wird (durch Abgießen, Abdekantieren oder einfaches Abfließenlassen). Dies wird beispielsweise in einem Behälter, gegebenenfalls unter Rühren, oder an dem in einem oder mehreren Rohren als Festbett angeordneten Katalysator ausgeführt. Diese letzte Methode empfiehlt sich besonders bei Großanlagen, in denen der Katalysator bereits in den Rohren des Reaktors vorliegt. Das Übergießen (Fluten) kann einmal oder mehrmals (mit der abgetrennten oder mit einer neu bereiteten Imprägnierflüssigkeit) durchgeführt werden.

Nach einer weiteren Arbeitsweise kann die Behandlung des Katalysators mit der Imprägnierflüssigkeit so erfolgen, daß ein Konzentrationsgefälle von 0,5 bis 20 mg/kg, vorzugsweise von 1 bis 15 mg/kg Cäsium und/oder Rubidium auf mindestens 2, vorzugsweise auf 5 bis 20 Meter Festbett — entgegen der Strömungsrichtung der umzusetzenden Gase — auf dem Katalysator erzeugt wird. In diesem Fall beträgt der mittlere Gehalt des Silber-Trägerkatalysators nach der Behandlung zweckmäßigerweise 10 bis 1000 mg/kg Cäsium und/oder Rubidium. Dabei können einzelne Teilstücke der gesamten als Festbett angeordneten Katalysatorteilchen durchaus niedrigere Konzentrationen als 10 und höhere Konzentrationen als 1000 mg/kg Cäsium und/oder Rubidium aufweisen. Andererseits sollen die Katalysatorteile mit der geringsten Menge aufgetragenen Cäsiums oder Rubidiums noch mindestens 1 mg/kg Cäsium und/oder Rubidium enthalten, da unterhalb dieser Konzentration ebenfalls eine vorteilhafte Wirkung nicht mehr erreicht wird. Solche Katalysatorteilchen verbrauchen unnötig einen weniger effektiven Raum im Festbett.

Ebenso soll die von den umzusetzenden Gasen durchströmte, mit Imprägnierflüssigkeit behandelte Katalysatorschicht im Festbett mindestens zwei Meter betragen. Nach oben ist die Länge der von den Gasen durchströmten Katalysatorschicht nur durch technisch-wirtschaftliche Erwägungen begrenzt; sie kann 40 Meter und mehr betragen, vorzugsweise wird eine Länge von 5 bis 20 Meter gewählt.

Für die Herstellung des beschriebenen Konzentrationsgefälles durch Behandlung des gewaschenen Katalysators mit Imprägnierflüssigkeit ist folgendes Verfahren vorzugsweise geeignet:

Mehrere getrennte Portionen des bereits gewaschenen Silberkatalysators werden mit Imprägnierlösungen benetzt, die voneinander verschiedene Konzentrationen innerhalb eines Bereiches von 0,003 bis 0,6 Gew.-%, bezogen auf die Lösung, an Cäsium und/oder Rubidium in Form der gewählten Verbindung(en) aufweisen, wobei jede Katalysatorportion etwa 3 bis 120 Minuten mit der Imprägnierlösung in Kontakt bleibt. Die so behandelten Katalysatorportionen werden in dem Reaktor so angeordnet, daß in Strömungsrichtung der an dem Katalyastor umzusetzenden Gase (Äthylen mit Sauerstoff oder Luft) die Cäsium- und/oder Rubidiumkonzentrationen der Impränierlösungen mit denen die Katalysatorportionen behandelt wurden, ständig zunimmt. Diese Zunahme der Cäsium- und/oder Rubidiumkonzentrationen der verschiedenen Imprägnierlösungen kann stetig oder unstetig verlaufen. Zweckmäßig wählt man eine etwa stetige Zunahme. Gute Ergebnisse werden erzielt mit einer Konzentrationszunahme, die 3 bis 30% der niedrigsten Konzentration oder der jeweils vorangegangenen Konzentration beträgt. Nach oben ist die Zahl der zu wählenden Katalysator-Portionen nur durch technischwirtschaftliche Erwägungen begrenzt. Im Prinzip können beliebig viele Katalysator-Portionen gewählt werden. Vorzugsweise werden 3 bis 20 Portionen mit ebensoviel Imprägnierlösungen verschiedener Konzentration behandelt. Wenn man dentechnischen Aufwand und Effekt vergleicht, werden besonders gute Ergebnisse mit 5 bis 15 Katalysator-Portionen erzielt. Diese Katalysator-Portionen können unter sich verschieden groß sein, vorzugsweise werden gleich große Portionen genommen.

Die Benetzung der Katalysator-Portionen mit den Imprägnierlösungen kann beispielsweise durch Aufsprühen oder Übergießen erfolgen. Als einfach zu handhaben und gut wirksam hat es sich erwiesen, die jeweilige Katalysator-Portion in ein Gefäß zu schütten und mit Imprägnierlösung bis etwas über die Standhöhe der Katalysatorteilchen aufzufüllen. Nach einer Einwirkungszeit von etwa 3 bis 120 Minuten, vorzugsweise 5 bis 20 Minuten, wird die überschüssige Imprägnierlösung abgetrennt.

Das Aufbringen von 1 bis 1000 Teilen, vorzugsweise 3 bis 500 Teilen Cäsium und/oder Rubidium je 1 Million Teile Katalysator auf den gewaschenen Katalysator kann auch in zwei oder mehr, vorzugsweise zwei bis fünf (zeitlich verschobenen) Schritten erfolgen, wobei der Katalysator nach jedem Behandlungsschritt wieder für die Direktoxidation von Äthylen zu Äthylenoxid mit molekularem Sauerstoff oder Luft eingesetzt wird. Die zeitliche Verschiebung zwischen den einzelnen Behandlungsschritten, das heißt die Zeitspanne, während der der Katalysator nach einer Behandlung wieder zur Direktoxidation eingesetzt worden ist, kann innerhalb weiter Grenzen variieren; sie liegt zweckmäßigerweise bei einer Woche bis zu mehreren Monaten, vorzugsweise bei 1 bis 20 Wochen, insbesondere bei 3 bis 5 Wochen. Nach oben ist die Grenze der Zeitspanne mehr nach technisch-wirtschaftlichen Gesichtspunkten begrenzt und die untere Grenze liegt zweckmäßigerweise nicht unter einer Stunde. Die Menge an Cäsium und/oder Rubidium, die pro Behandlungsschritt auf den gewaschenen Katalysator aufgebracht wird, kann innerhalb des Bereiches 1 bis 1000 mg/kg, vorzugsweise 3 bis 500 mg/kg variiert werden. In der Regel wird man beim ersten Behandlungsschritt mindestens 1 mg/kg, vorzugsweise 3 bis 100 mg/kg auf den Katalysator aufbringen und bei den folgenden Behandlungsschritten jeweils die gleiche oder eine Teilmenge davon.

Nach dem Behandeln des gewaschenen Katalysators mit Impräg-nierflüssigkeit (nach einer der vorhin beschriebenen Methoden), ist es zweckmäßig, wenn die nach Abtrennen des größten Teils der Imprägnierflüssigkeit am Katalysator gegebenenfalls verbliebenen Flüssigkeitsreste entfernt werden, was nach einem der oben beschriebenen Trocknungsverfahren erfolgen kann. Die dabei gegebenenfalls angewendeten Temperaturen richten sich, ähnlich wie dort, nach der zu entfernenden (verdampfenden) Imprägnierflüssigkeit und liegen bei 50 bis 250°C, vorzugsweise bei 50 bis 150°C, insbesondere bei 70 bis 120°C.

Das erfindungsgemäße Verfahren ist unabhängig von der Art (beispielsweise Zusammensetzung, Aufbau, Struktur) des Silberkatalysators selbst. Jeder für die Direktoxidation von Äthylen zu Äthylenoxid mit molecularem Sauerstoff oder Luft geeignete Silberkatalysator kommt für das erfindungsgemäße Verfahren in Betracht.

Silberkatalysatoren für die Direktoxidation von Äthylen zu Äthylenoxid mit molekularem Sauerstoff oder Luft sind, ebenso wie die Direktoxidation selbst, in der Literatur eingehend beschrieben, beispielsweise in den folgenden amerikanischen Patentschriften: 2 615 899, 3 899 445 und 3 962 136.

Die in Rede stehenden Silberkatalysatoren bestehen im allgemeinen aus 1 bis 40 Gew.-% (bezogen auf den gesamten Katalysator) an Silber auf einem Trägermaterial und gegebenenfalls aus mehr oder weniger großen Mengen verschiedenster Promotoren oder Koaktivatoren. Dabei ist das Silber als Metall auf den inneren und äußeren Oberflächen des vorzugsweise porösen Trägermaterials niedergeschlagen und möglichst gleichmäßig über dessen gesamte Oberfläche verteilt. Die Morphologie des auf dem Trägermaterial aufgebrachten Silbers kann innerhalb weiter Grenzen variieren. Im allgemeinen hat es die Gestalt von kugelförmigen Teilchen mit einem Durchmesser von 0,01 bis 10 $\mu$. Das Trägermaterial besteht zweckmäßigerweise aus porösem, hitzbeständigen und unter den bei der Direktoxidation von Äthylen auftretenden Bedingungen inert bleibenden Materialien.

Beispiele für solche Materialien sind Aluminiumverbindungen, vorzugsweise Aluminiumoxide verschiedenster Struktur, Magnesiumoxide, Kieselgur, Bimsstein, Siliciumdioxid, Siliciumcarbid, Tone, Korund, Zeolithe, Metalloxide, und ähnliche. Ein besonders bevorzugtes Trägermaterial sind $\alpha$-Aluminiumoxide, da sie einen weitgehend gleichmäßigen Porendurchmesser aufweisen. Sie werden charakterisiert insbesondere durch die spezifische Oberfläche ($m^2$/g), das spezifische Porenvolumen ($cm^3$/g) und den mittleren Porendurchmesser ($\mu$). Die Trägermaterialien werden in der Regel in Form von Granulaten, Kugeln, Stückchen, Ringen oder dgl. eingesetzt.

Das erfindungsgemäße Verfahren bezieht sich auf gebrauchte Silberkatalysatoren. Der Ausdruck "gebraucht" bedeutet in diesem Zusammenhang, daß der Katalysator zur Umsetzung von Äthylen zu Äthylenoxid mit molekularem Sauerstoff oder Luft bereits eingesetzt war, wobei es belanglos ist, ob dabei seine ursprüngliche Selektivität nachgelassen hat oder nicht. Die Zeit, während der der Katalysator für die Oxidation von Äthylen zu Äthylenoxid vor der erfindungsgemäßen Behandlung in Gebrauch war, kann innerhalb weiter Grenzen variieren; sie kann von wenigen Wochen (1 bis 3) bis mehrere Jahre (1 bis 10) und darüber schwanken. Dabei kann der Katalysator in seiner Wirksamkeit nachgelassen, das heißt in der Selektivität abgenommen haben (was in der Regel nach längerer Gebrauchszeit eintritt), er kann auch die ursprüngliche Selektivität beibehalten haben.

Mit der erfindungsgemäßen Kombination von Waschen und Aufbringen von Cäsium und/oder Rubidium gelingt es also, Silber-Trägerkatalysatoren, die bereits für die Direktoxidation von Äthylen mit molekularem Sauerstoff oder Luft in Gebrauch waren, in ihrer Wirksamkeit erheblich zu verbessern. Die Wirksamkeit eines Katalysators kann ausgedrückt werden als Umsatz (in %) von Äthylen bei gegebener Temperatur oder als molares Verhältnis von in Äthylenoxid umgesetztes Äthylen, das ist seine Selektivität. Ein Katalysator ist um so wirksamer, je mehr Äthylen bei einer bestimmten Temperatur umgesetzt wird, je höher die Selektivität bei einem bestimmten Umsatz ist und je niedriger die Temperatur zur Erzielung eines bestimmten Umsatzes ist.

Mit dem erfindungsgemäßen Verfahren wird nicht nur die Selektivität von gebrauchten Silber-Trägerkatalysatoren sondern auch der Umsatz wesentlich gesteigert. Darüberhinaus ist es auch möglich, bei erfindungsgemäß behandelten Katalysatoren die Reaktionstemperatur abzusenken — bei gleichem oder sogar erhöhtem Umsatz —. Dies ist deshalb besonders bedeutungsvoll, weil mit niedrigerer Reaktionstemperatur die Bildung unerwünschter Nebenprodukte wie Kohlendioxid, Formaldehyd, Acetaldehyd, stark zurückgeht. Angesichts der großen Mengen Äthylenoxid, die nach dem Äthylen-Oxidations-verfahren produziert werden, kommt einer Ausbeutesteigerung auch nur um wenige Prozent oder sogar um Zehntelprozent erhebliche wirtschaftliche Bedeutung zu. Was das erfindungs-gemäße Verfahren ferner auszeichnet, ist der Umstand, daß es in den üblichen Großproduktions-Anlagen (mit den handels-üblichen Silber-Trägerkatalysatoren) ohne nennenswerten zusätzlichen Aufwand an Energie, Investitionen und Material durchgeführt werden kann.

Die Erfindung wird an Beispielen näher erläutert.

Die folgenden Beispiele und Vergleichsbeispiele werden in einem Versuchsreaktor aus einem senkrecht stehenden Reaktionsrohr aus Chromvanadiumstahl mit einer lichten Weite von 30 mm und einer Länge von 300 mm durchgeführt. Das mit einem Mantel versehene Reaktionsrohr wird mit heißem Öl, das durch den Mantel strömt, beheizt. Das Reaktionsrohr ist bis zu einer Höhe von 200 mm mit $\alpha$-$Al_2O_3$-Pellets gefüllt; diese Füllung dient zur Vorhbeizung des Einsatzgases. Auf der inerten Füllung liegt der zu prüfende Katalysator. Das Einsatzgas tritt unten (drucklos) in das Reaktionsrohr ein und verläßt es am Kopf.

Das eingesetzte Gasgemisch besteht aus

| | |
|---|---|
| C₂H₄ | 28 Vol.-% |
| CH₄ | 53 Vol.-% |
| O₂ | 8 Vol.-%<br>Gasgemisch I |
| CO₂ | 5 Vol.-% |
| N₂ | 6 Vol.-% |
| Vinylchlorid | 0,0002 Vol.-%<br>(Inhibitor) |

oder aus

| | |
|---|---|
| C₂H₄ | 4 Vol.-% |
| O₂ | 5 Vol.-%<br>Gasgemisch II |
| CO₂ | 4 Vol.-% |
| N₂ | 87 Vol.-% |

Die Raum-Zeit-Geschwindigkeit beträgt:

$$250 \cdot \frac{\text{Raumteile Gas}}{\text{Stunde} \cdot \text{Raumteile Katalysator}}$$

Das am Reaktorausgang austretende Gas wird gaschromatographisch analysiert und Umsatz und Selektivität berechnet. Die Temperatur des Wärmeträgermediums wird so lange variiert, bis ein konstanter Äthylenumsatz von 7% bei Gasgemisch I, bzw. von 35% bei Gasgemisch II erzielt wird.

Die Laufzeit der Versuche wird so gewählt, daß am Ende keine Veränderung der Meßwerte mehr erfolgt. Das ist normalerweise bei einer Laufzeit von 200 Stunden der Fall.

Für die Versuche werden handelsübliche Silber-Trägerkatalysatoren eingesetzt. Sie bestehen aus 10% Silber (Teilchendurchmesser 1 bis 5 μ) auf α-Al₂O₃ als Trägermaterial, das die Form von Ringkörpern mit einer Länge von 8 mm, einem äußeren Durchmesser von 8 mm und einem inneren Durchmesser von 2 mm — Katalysator I — oder das die Form von Zylindern mit einem Durchmesser und einer Höhe von etwa 5 mm — Katalysator II — besitzt; die spezifische Oberfläche liegt bei 0,1 bis 0,5 m²/g.

Der auf dem Katalysator aufgebrachte Cäsium- und/oder Rubidiumgehalt wird durch Atomabsorptionsspektroskopie bestimmt (siehe Monographie: "Atomabsorptionsspektroskopie", Bernhard Wells Verlag Chemie 1972, Seite 114 ff.). Die Bestimmung wird in einer Luft-Acetylenflamme durchgeführt und die Atomabsorption in Emission gemessen.

### Beispiel 1

50 g des oben beschriebenen handelsüblichen Katalysators II, der 10 Jahre zur Herstellung von Äthylenoxid durch Direktoxidation von Äthylen mit Sauerstoff eingesetzt war, werden in einem 100 ml Erlenmeyerkolben mit 50 ml Methanol bei 25°C überschüttet und so 5 Stunden lang stehen gelassen. Nach dem Abdekantieren des Methanols wird ein zweitesmal mit 50 ml frischem Methanol überschüttet und nach 5 Stunden das Methanol wiederum abgegossen. Nach dieser Methanol-Wäsche wird der von Methanol noch feuchte Katalysator, ebenfalls im 100 mi-Kolben, mit einer Lösung aus 50 ml Methanol und 0,0205 g Cäsiumnitrat übergossen und 1 Stunde stehen gelasen. Anschließend wird die überschüssige Tränklösung abgegossen und der Katalysator 1 Stunde bei 110°C im Trockenschrank getrocknet. Die Cäsiumkonzentration auf dem Katalysator beträgt 90 mg/kg.

Der so behandelte Katalysator wird nun in den Versuchsreaktor eingefüllt und in der beschriebenen Weise mit Gasgemisch I getestet: Die Selektivität ist von vorher 69% auf nunmehr 76% angestiegen.

### Vergleichsbeispiel 1

Es wird wie in Beispiel 1 vorgegangen, wobei jedoch die Methanol-Wäsche weggelassen wird.

Es ergibt sich eine Steigerung der Selektivität von 69% auf 74,5%.

### Beispiel 2

50 g des in Beispiel 1 benutzten Katalysators werden, wie oben beschrieben, in einem 100 ml Erlenmeyerkolben dreimal mit einer Waschflüssigkeit aus Isopropanol und 20 Gew.-% Wasser, bezogen auf Gesamtmischung, gewaschen. Nach dem letzten Abdekantieren wird der gewaschene Katalysator mit einer Lösung aus 50 ml Methanol und 0,020 g Cäsiumcarbonat wie in Beispiel 1 behandelt und anschließend getestet (Cäsiumkonzentration auf Katalysator: 91 mg/kg).

Die Selektivität ist von vorher 69% auf 76% gestiegen.

### Vergleichsbeispiel 2

Es wird wie in Beispiel 2 vorgegangen, wobei jedoch das Waschen des Katalysators nicht vorgenommen wird. Es ergibt sich eine Steigerung der Selektivität von 69% auf 74%.

### Beispiel 3

100 g des oben beschriebenen Silberkatalysators I, der 5 Monate in einer Versuchsanlage zur Herstellung von Äthylenoxid aus Äthylen und Sauerstoff eingesetzt war, werden in ein Glasrohr von 20 mm lichter Weite und 500 mm Länge gegeben. Nun werden bei Raumtemperatur 500 g Benzol, das 10 Gew.-%, bezogen auf Gesamtmischung, Dioxan enthält, im Laufe einer Stunde (von unten nach oben) über den Katalysator gepumpt. Nach dem Ablaufenlassen der Waschflüssigkeit wird eine Lösung von 0,042 g Cäsiumacetat in 100 g Me-

Es ergibt sich eine Steigerung der Selektivität von 68% auf 72,5%.

### Beispiel 4

100 g des oben beschriebenen, handelsüblichen Silberkatalysators I, der 4 Jahre zur Herstellung von Äthylenoxid aus Äthylen und Sauerstoff eingesetzt war, werden in einem 200 ml Erlenmeyerkolben mit 100 g Aceton bei 25°C überschüttet und so 1 Stunde stehen gelassen. Nach dem Abdekantieren der Waschflüssigkeit wird ein zweitesmal mit 100 g frischem Aceton überschüttet und nach 1 Stunde Standzeit abgegossen.

Nach dieser Aceton-Wäsche wird der Katalysator in 5 gleiche Portionen geteilt. Eine Portion wird in einem 50 ml Erlenmeyerkolben mit 30 ml einer äthanolischen Lösung, die 285 mg Cäsiumsulfat pro kg Äthanol enthält, übergossen und 1 Stunde stehen gelassen. Nach dem Abgießen der überschüssigen Tränklösung wird der Katalysator 1 Stunde im Trockenschrank bei 110°C getrocknet. Die restlichen 4 Portionen werden mit einer Lösung von 333, 381, 428 bzw. 476 mg Cäsiumsulfat pro kg Äthanol, wie oben beschrieben getränkt und getrocknet.

Die behandelten Katalysator-Portionen werden nun so in den Versuchreaktor eingefüllt, daß die Portion mit dem geringsten Cäsiumgehalt mit dem frischen Äthylen-Sauerstoffgemisch — Gasgemisch I — zuerst in Berührung kommt, also am Gaseingang liegt, und die weiteren Portionen auf diese mit jeweils steigendem Cäsiumgehalt folgen.

Die Selektivität steigt von 66% auf 75%.

### Vergleichsbeispiel 4

Es wird wie in Beispiel 4 vorgegangen, wobei jedoch die Acetonwäsche weggelassen wird.

Die Selektivität steigt von 66 auf nur 73,5%.

### Beispiel 5

100 g des oben beschriebenen handelsüblichen Silberkatalysators I, der 12 Monate in einer Versuchsanlage zur Herstellung von Äthylenoxid aus Luft und Äthylen eingesetzt war, wird in ein Glasrohr von 20 mm lichter Weite und 500 mm Länge gegeben. Mittels einer Pumpe werden 400 ml Petroläther (Siedepunkt 40 bis 70°C) bei 25°C (von unten nach oben) über den Katalysator gepumpt. Die ersten 100 ml Petroläther werden nach dem Verlassen des Bettes verworfen und die weiteren 300 ml 1 Stunde lang mit 0,5 l pro Stunde im Kreis gepumpt.

Anschließend läßt man den Petroläther ablaufen und trocknet 1 Stunde mit 100 l Stickstoff pro Stunde, wobei der Katalysator mit Hilfe eines Wärmeträgermediums im Doppelmantel des Glasrohres auf 60°C erwärmt wird. Nach dem Abkühlen auf 25°C wird der Katalysator im Glasrohr mit einer methanolischen Lösung, die 500 mg Cäsiumnitrat pro kg Methanol enthält, übergossen. Diese Tränklosung läßt man nach 1 Stunde ablaufen und trocknet den Katalysator durch Überleiten von 100 l Stickstoff bei 80°C während 1 Stunde. Der Katalysator wird nun in den Versuchsreaktor eingefüllt und auf beschriebene Weise mit Gasgemisch II getestet. Es ergibt sich eine Selektivitätssteigerung von 60 auf 67%.

### Vergleichsbeispiel

Es wird wie in Beispiel 5 vorgegangen, wobei jedoch die Petrolätherwäsche weggelassen wird.

Die Selektivität steigt von 60% auf 65%.

### Patentansprüche

1. Verfahren zur Verbesserung der Wirksamkeit von Silber-Trägerkatalysatoren für die Direktoxidation von Äthylen zu Äthylenoxid mit molekularem Sauerstoff oder Luft, wobei auf den für die Direktoxidation gebrauchten Katalysator 1 bis 1000 Teile, je 1 Million Teile Katalysator, Cäsium, Rubidium oder eine Mischung davon aufgebracht wird, dadurch gekennzeichnet, daß vor dem Aufbringen der gebrauchte Katalysator mit einer inerten organischen Flüssigkeit gewaschen wird.

2. Verfahren nach Anspruch 1, dadurch gekennzeichnet, daß die inerte organische Flüssigkeit einen Siedepunkt von 20 bis 150°C aufreist.

3. Verfahren nach Anspruch 1, dadurch gekennzeichnet, daß als inerte organische Flüssigkeit ein aliphatischer Kohlenwasserstoff mit 5 bis 10 C-Atomen, Petroläther, Cyclohexan, Benzol, ein aliphatischer Äther mit 4 bis 8 C-Atomen, Dioxan, Tetrahydrofuran, ein aliphatisches Keton mit 3 bis 6 C-Atomen, ein aliphatischer Alkohol mit 1 bis 3 C-Atomen oder eine Mischung davon eingesetzt wird.

4. Verfahren nach Anspruch 1, dadurch gekennzeichnet, daß das Waschen in einem Gefäß durch 1- bis 5 maliges Übergießen des Katalysators mit Waschflüssigkeit, Stehenlassen in der Waschflüssigkeit für 1 bis 5 Stunden und Abdekantieren der Waschflüssigkeit erfolgt.

5. Verfahren nach Anspruch 1, dadurch gekennzeichnet, daß das Waschen durch Fluten des Katalysators erfolgt.

6. Verfahren nach einem der Ansprüche 1 bis 5, dadurch gekennzeichnet, daß nach dem Waschen auf den Katalysator 3 bis 500 ppm Cäsium und/oder Rubidium aufgebracht werden.

### Revendications

1. Procédé pour améliorer l'activité de catalyseurs à l'argent sur un support pour l'oxydation directe de l'éthylène en oxyde d'éthylène par l'oxygène moléculaire ou l'air, dans lequel on dépose sur le catalyseur usé, par million de parties de celui-ci, 1 à 1000 parties de césium ou de rubidium ou d'un mélange des deux, procédé caractérisé en ce qu'avant d'effecteur ce dépôt on lave le catalyseur usé avec un liquide organique inerte.

2. Procédé selon la revendication 1, dans lequel le liquide organique inerte a un point d'ébullition compris entre 20 et 150°C.

3. Procédé selon la revendication 1, dans lequel on utilise comme liquide organique inerte un hydrocarbure aliphatique en $C_5$ à $C_{10}$, de l'éther de pétrole, du cyclohexane, du benzène, un éther aliphatique en $C_4$ à $C_8$, le dioxanne, le tétrahydrofuranne, une cétone aliphatique en $C_3$ à $C_6$, un alcool aliphatique en $C_1$ à $C_3$ ou un mélange de plusieurs de ces liquides.

4. Procédé selon la revendication 1, dans lequel on effectue le lavage dans un récipient en recouvrant 1 à 5 fois le catalyseur avec le liquide de lavage, en le laissant chaque fois au repos dans le liquide pendant 1 à 5 heures, puis en éliminant le liquide par décantation.

5. Procédé selon la revendication 1, dans lequel le lavage se fait par noyage due catalyseur avec circulation du liquide.

6. Procédé selon 1'une quelconque des revendications 1 à 5, dans lequel après avoir lavé le catalyseur on y déposé 3 à 500 ppm césium et/ou de rubidium.

**Claims**

1. Process to improve the activity of supported silver catalysts for the direct oxidation of ethylene to ethylene oxide with molecular oxygen or air, whereby on the catalyst used for the direct oxidation are applied from 1 to 1,000 parts, per 1 million part of catalyst, of cesium, rubidium or a mixture thereof, which comprises washing the used catalyst with an inert organic liquid prior to said application.

2. A process as claimed in claim 1, wherein the inert organic liquid has a boiling point of from 20 to 150°C.

3. A process as claimed in claim 1, wherein the inert organic liquid is an aliphatic hydrocarbon having from 5 to 10 carbon atoms, petroleum ether, cyclohexane, benzene, an aliphatic ether having from 4 to 8 carbon atoms, dioxane, tetrahydrofurane, an aliphatic ketone having from 3 to 6 carbon atoms, an aliphatic alcohol having from 1 to 3 carbon atoms, or a mixture thereof.

4. A process as claimed in claim 1, wherein the catalyst is washed in a container by pouring the washing liquid thereover 1 to 5 times, the mixture of washing liquid and catalyst is left to stand for 1 to 5 hours and the washing liquid is then decanted.

5. A process as claimed in claim 1, wherein the catalyst is washed by flooding.

6. A process as claimed in claims 1 to 5, wherein 3 to 500 ppm of cesium, rubidium, or mixture thereof are applied to the catalyst after washing.